# EUROPEAN PATENT APPLICATION

(11) **EP 1 547 517 A1**
(43) Date of publication of application: **29.06.2005**
(21) Application number: 04030312.5
(22) Date of filing: 21.12.2004
(51) Int. Cl.: A61B 5/0404

(54) **Portable electrocardiograph**

(30) Priority: 26.12.2003 JP 2003434557
(71) Applicant: Omron Healthcare Co., Ltd., Kyoto 615-0084 (JP)
(72) Inventor: Umeda, Masahiro Omron Healthcare Co., Ltd., Kyoto-shi Kyoto 615-0084 (JP); Yamamoto, Norihito Omron Healthcare Co., Ltd., Kyoto-shi Kyoto 615-0084 (JP); Ishida, Junichi Omron Healthcare Co., Ltd., Kyoto-shi Kyoto 615-0084 (JP); Tanabe, Kazuhisa Omron Healthcare Co., Ltd., Kyoto-shi Kyoto 615-0084 (JP); Moroki, Yoko Omron Healthcare Co., Ltd., Kyoto-shi Kyoto 615-0084 (JP)
(74) Representative: Kilian, Helmut, Dr.

(57) **Abstract**

A portable electrocardiograph (100A) includes a first electrode (121) on an outer surface of a device main body (110) and a second electrode (122) drawn outside the device main body (110) via a connection cable (181). The first electrode (121) and the second electrode (122) are brought into contact with a body surface. Then, a potential difference between the first and second electrodes is measured in order to measure an electrocardiographic waveform. In this manner, a noise caused by a myoelectric potential produced in muscles other than cardiac muscle is not superposed on the electrocardiographic waveform, and the electrocardiographic waveform can precisely be measured in a stable manner.

## Description

### BACKGROUND OF THE INVENTION

### Field of the Invention

The present invention relates to a portable electrocardiograph capable of easily measuring and storing electrocardiographic waveforms.

### Description of the Background Art

Generally, for diagnosing ischemic cardiomyopathies such as angina pectoris and myocardial infarction, an electrocardiogram of a patient is used. Known electrocardiographs used for measuring electrocardiographic waveforms include a stationary electrocardiograph and a portable electrocardiograph.

The stationary electrocardiograph is installed in a medical institution such as a hospital and is used in such a manner that electrodes are attached to a body of a patient who lies on a bed or the like to measure electrocardiographic waveforms. Measurement of the electrocardiographic waveforms by using the stationary electrocardiograph is advantageous in that various electrocardiographic waveforms (such as P waveform, QRS waveform, or ST waveform) can precisely be measured. On the other hand, there is a possibility that an abnormal waveform is not necessarily reproduced at the time of measurement, and a symptom of a patient sometimes cannot properly be observed at the time of diagnosis by a physician.

Portable electrocardiographs are roughly divided into a Holter electrocardiograph for continuously measuring and storing electrocardiographic waveforms while electrodes are attached to the body of the patient who lives daily life for one day to several days, and an event-type electrocardiograph for measuring and storing electrocardiographic waveforms when a subjective symptom to be measured such as palpitation or pant occurs.

In the Holter electrocardiograph, a state where the electrodes are attached to the body of the patient should be maintained for a long time. Accordingly, an adhesion-type electrode is normally adopted as each of a plurality of measurement electrodes. The adhesion-type electrode is implemented by applying a conductive adhesive on a surface of the measurement electrode. The electrode is attached to the body by means of an adhesive layer. It is noted that the adhesion-type electrode is electrically connected to a device main body via a connection cable extending from an electrode body.

In the Holter electrocardiograph, abnormal waveforms can reliably be measured. On the other hand, since the state where electrodes are attached to the patient should be maintained for one day to several days, the patient may feel unpleasant or pain.

Known event-type portable electrocardiographs include an electrocardiograph of a type in which an electrode for measuring electrocardiographic waveforms is always in contact with a prescribed site of the body and an electrocardiograph of a type in which a subject himself/herself contacts an electrode with the body when a subjective symptom to be measured occurs.

In the former event-type portable electrocardiograph, like the Holter portable electrocardiograph, the state where the electrode is always in contact with the body has to be maintained. As such, the adhesion-type electrode is employed. Here, the subject suffers from unpleasant feeling or pain, as in the case of the Holter portable electrocardiograph described above. In contrast, in the latter event-type portable electrocardiograph, the electrode should be brought into contact with the body only when necessary. Therefore, the event-type portable electrocardiograph is very easy to use for the subject.

As the latter event-type portable electrocardiographs, various electrocardiographs structured such that an electrode is provided on an outer surface of the device main body have been proposed.

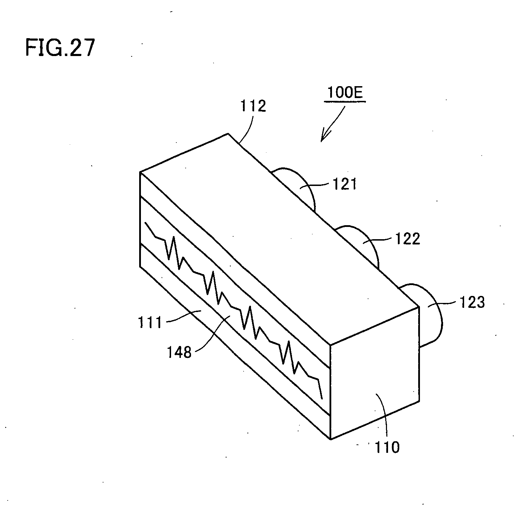

For example, as shown in Fig. 27, Japanese Patent Laying-Open No. 61-41438 discloses a portable electrocardiograph 100E structured such that a display unit 148 is provided on a front face 111 of a device main body 110 and three electrodes 121, 122 and 123 to be attached to the body are provided on a rear face 112. Measurement is carried out by bringing three electrodes 121, 122 and 123 into contact with a chest of a subject.

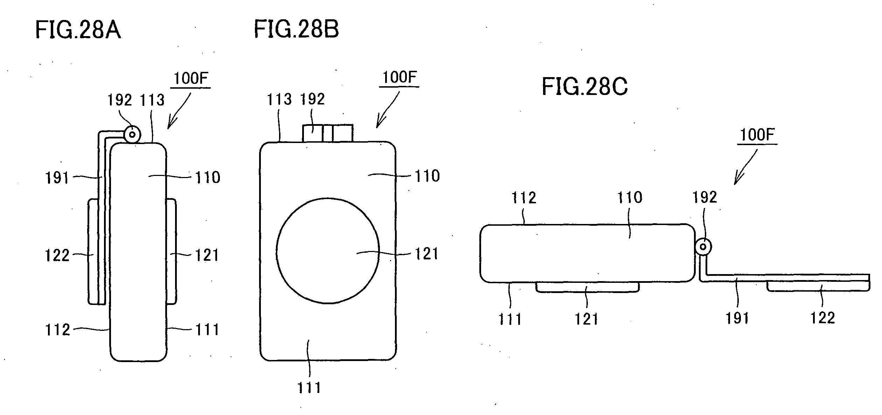

As shown in Figs. 28A and 28B, Japanese Utility Model Laying-Open No. 3-91304 discloses a portable electrocardiograph 100F structured such that electrode 121 is provided on front face 111 of device main body 110, a supporting member 191 extending from the top of device main body 110 toward rear face 112 of device main body 110 is attached to a top face 113 of device main body 110 with a hinge 192, and electrode 122 is provided on a surface of supporting member 191. At the time of measurement, supporting member 191 is pivoted to open the electrocardiograph (see Fig. 28C), so that an electrode formation face of supporting member 191 and front face 111 of device main body 110 are located on substantially the same plane, and a electrocardiographic waveform is measured in a state where two electrodes 121 and 122 are brought into contact with the chest of the subject.

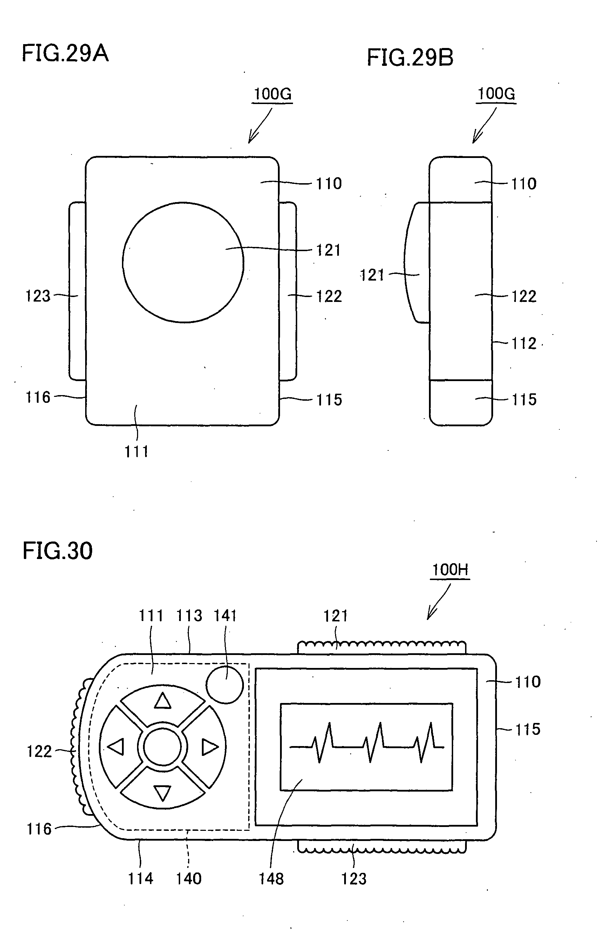

As shown in Figs. 29A and 29B, Japanese Utility Model Laying-Open No. 3-91305 discloses a portable electrocardiograph 100G structured such that electrode 121 made of an electroconductive rubber is provided on front face 111 of device main body 110 and electrodes 122 and 123 are provided on a right side face 115 and a left side face 116 of device main body 110 respectively, At the time of measurement, the subject holds device main body 110 from rear face 112 side thereof so as to touch electrodes 122 and 123 provided on both side faces 115 and 116, and brings electrode 121 provided on front face 111 into contact with the chest of the subject so as to measure an electrocardiographic waveform.

As shown in Fig. 30, Japanese Patent Laying-Open No. 2003-144403 discloses a portable electrocardiograph 100H structured such that a negative electrode 121 and an indifferent electrode 123 are provided on top face 113 and a bottom face 114 respectively, that are opposing surfaces of device main body 110 having a substantially rectangular parallelepiped shape, and a positive electrode 122 is provided on left side face 116 which is a curved surface adjacent to the surfaces on which negative electrode 121 and indifferent electrode 123 are provided. On front face 111 of device main body 110, display unit 148 for displaying a measurement result as well as an operation button portion 140 where various operation buttons represented by a power button 141 for turning on power are disposed are provided. At the time of measurement, the subject himself/herself holds negative electrode 121 and indifferent electrode 123 provided on top face 113 and bottom face 114 respectively from the rear side of device main body 110 with his/her right hand, and brings electrode 122 provided on left side face 116 of device main body 110 into contact with his/her chest so as to measure an electrocardiographic waveform.

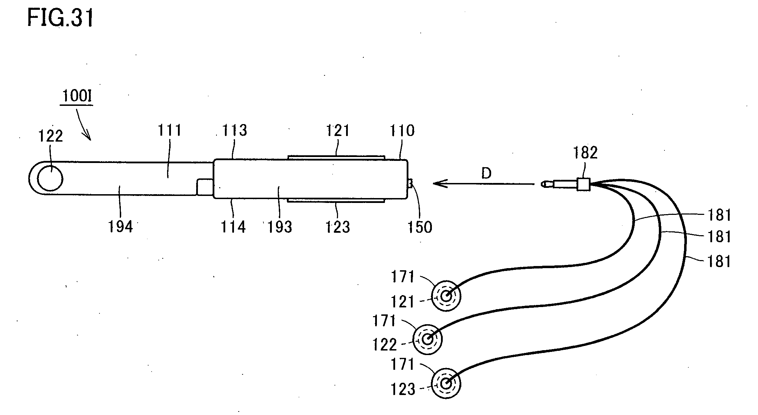

Meanwhile, in Japanese Patent Laying-Open No. 9-56686 discloses a portable electrocardiograph structured so as to be capable of switching between Holter-type and event-type depending on a purpose of use. As shown in Fig. 31, in a portable electrocardiograph 100I disclosed in Japanese Patent Laying-Open No. 9-56686, device main body 110 is divided into a main body portion 193 and an arm portion 194 connected to each other by a hinge mechanism. On top face 113 and bottom face 114 that are opposing surfaces of main body portion 193, one measurement electrode 121 and indifferent electrode 123 are provided respectively. The other measurement electrode 122 is provided at a tip end of arm portion 194 on front face 111 of device main body 110. In addition, a jack 150 for receiving a connector 182 of connection cables 181 extending from attachment portions 171 of the adhesion-type electrodes (a pair of measurement electrodes 121 and 122 and indifferent electrode 123) is provided in a prescribed position of main body portion 193.

When this electrocardiograph is used as the event-type electrocardiograph, the adhesion-type electrodes are disconnected from device main body 110. Then, one measurement electrode 121 and indifferent electrode 123 are brought into contact with the body by holding main body portion 193 with the right hand, and the other measurement electrode 122 provided at the tip end of arm portion 194 is pressed against the chest. On the other hand, when the electrocardiograph is used as the Holter-type portable electrocardiograph, connector 182 of connection cables 181 extending from the adhesion-type electrodes is inserted in jack 150 provided in device main body 110 in a direction shown with an arrow D in the drawing, and the adhesion-type electrode is attached to a prescribed site of the chest. In a state where connector 182 is inserted in jack 150, measurement electrodes 121 and 122 as well as indifferent electrode 123 provided on an outer surface of device main body 110 are electrically disconnected from a circuit provided inside the device main body.

In such a portable electrocardiograph as disclosed in each document above, the contact portion between the electrode provided on the outer surface of a housing and the body should be kept stable during a measurement period of several tens of seconds. Unless stable contact is maintained, the measured waveform is disturbed, by variation in a contact area between the electrode and the body, and the electrocardiographic waveform cannot precisely be measured in a stable manner.

The conventional portable electrocardiograph described above is intended to maintain contact between the electrode and the body by pressing a hand holding the electrocardiograph main body against abdomen or the like of the body at the time of measurement, and by fixing that hand. More specifically, a wrist, a forearm, an elbow or the like of the holding arm is pressed against the body, thereby preventing the holding hand from being moved during measurement and maintaining stable contact between the electrode and the body. This point will be described in more detailed manner with reference to portable electrocardiograph 100H disclosed in Japanese Patent Laying-Open No. 2003-144403.

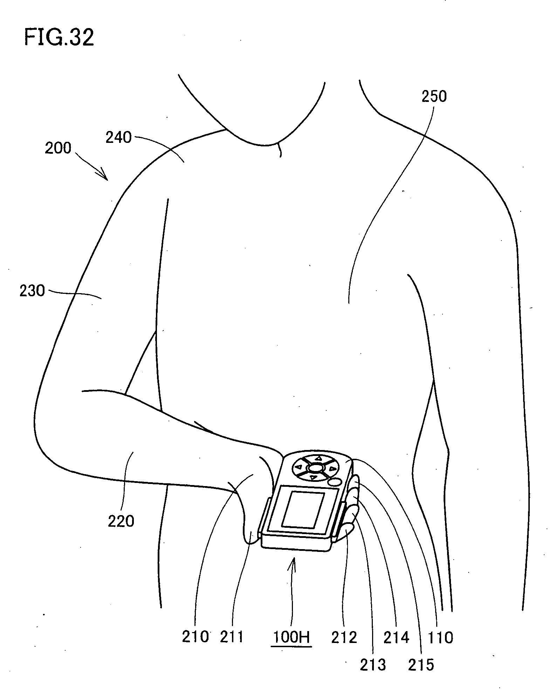

Fig. 32 is a perspective view of a measurement posture to be taken by the subject at the time of measuring an electrocardiographic waveform using portable electrocardiograph 100H disclosed in Japanese Patent Laying-Open No. 2003-144403. As shown in Fig. 32, during measurement, a subject 200 presses a wrist portion of forearm 220 against the right side of the body while holding portable electrocardiograph 100H with his/her right hand 210, and also brings positive electrode 122 provided on left side face 116 of device main body 110 of portable electrocardiograph 100H into direct contact with the skin of a lower left portion of a chest 250. While maintaining this state for several tens of seconds, an electrocardiographic waveform is measured.

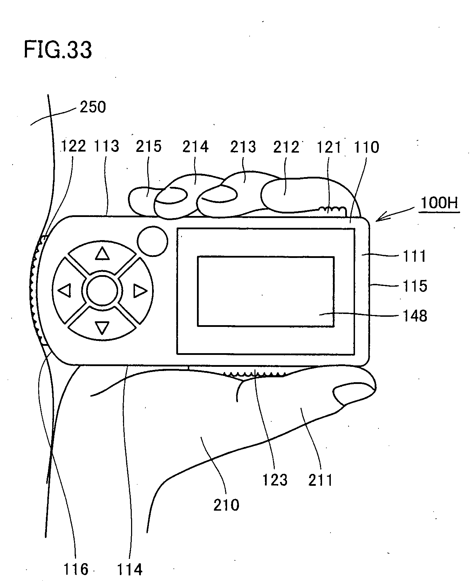

Fig. 33 shows a state where portable electrocardiograph 100H is held with right hand 210. As shown in Fig. 33, when subject 200 takes the measurement posture as shown in Fig. 32, he/she holds device main body 110 while covering the rear face side of device main body 110 with his/her palm, such that front face 111 of portable electrocardiograph 100H faces upward. Device main body 110 is held with a forefinger 212, a middle finger 213, a ring finger 214, and a little finger 215 of right hand 210 being lightly bent, so that any or all of the fingers come into contact with negative electrode 121 provided on top face 113 of device main body 110. A thumb 211 extends along bottom face 114 of device main body 110 and comes in contact with indifferent electrode 123 provided on bottom face 114, so as to hold device main body 110. Then, the wrist portion of right hand 210 is pressed against the right side of the body, and right hand 210 is fixed such that positive electrode 122 formed on left side face 116 of device main body 110 is not apart from the body.

In measuring the electrocardiographic waveform in such a measurement posture, if the wrist portion of the right arm is not covered with clothing or the like, the right arm and the right side of the body are in direct contact with each other. Consequently, a measurement circuit (an electric circuit formed in the body from the positive electrode to the negative electrode) is short-circuited in this portion. In such a case, the measurement circuit no longer crosses over the heart, resulting in failure in precise measurement of the electrocardiographic waveform.

Even if the wrist portion of the right arm is covered with clothing or the like, in a state where the portable electrocardiograph is held with the right hand and pressed against the right side of the body, the subject has to put strength in his/her right arm in order to hold the portable electrocardiograph. Here, muscles in the right arm are under a tension, resulting in generation of a myoelectric potential. When the myoelectric potential is produced in the right arm forming a part of the measurement circuit, the myoelectric potential is in turn superposed as noise on the electrocardiographic waveform to be measured. In particular with regard to elderly people who have less flexibility of the body, the myoelectric potential produced when the measurement posture described above is taken is high, which will considerably interfere precise measurement of the electrocardiographic waveform.

A variety of techniques (as disclosed in Japanese Patent Laying-Open Nos. 2000-14653, 2001-346771, 61-206428, and the like) have conventionally been developed in order to solve the above-described problems. The technique disclosed in each document, however, is directed to removal with a filter of noise as result of a myoelectric potential component produced in muscles other than cardiac muscle and mixed in measurement data. Unless complete removal of the noise is achieved, precise electrocardiographic data cannot be obtained. On the other hand, complete removal of the noise is substantially impossible, and it has been extremely difficult to accurately and precisely measure each characteristic waveform in the electrocardiographic waveforms (such as P waveform, QRS waveform, ST waveform, or the like) even with these techniques.

### SUMMARY OF THE INVENTION

An object of the present invention is to provide a portable electrocardiograph capable of precisely measuring an electrocardiographic waveform in a stable manner, without noise caused by a myoelectric potential produced in muscles other than cardiac muscle being superposed on the electrocardiographic waveform.

The inventors have conceived that superposition on the electrocardiographic waveform of the noise caused by the myoelectric potential produced in muscles other than cardiac muscle can be avoided by measuring the electrocardiographic waveform while a measurement posture preventing muscles in the right arm from being under tension is taken, and have found a measurement posture completely different from a posture taken in using a conventional portable electrocardiograph. More specifically, the inventors have found that superposition on the electrocardiographic waveform of the noise caused by the myoelectric potential produced in muscles in the right arm can considerably be suppressed by measuring the electrocardiographic waveform when such a posture that the right arm is supported by some kind of base or such a posture that the subject has his/her right arm vertically hang down is taken, thereby attaining precise measurement of the electrocardiographic waveform in a stable manner. The inventors have studied and developed a portable electrocardiograph allowing such a measurement posture, and finally completed the present invention.

A portable electrocardiograph according to the present invention measures an electrocardiographic waveform by measuring a potential difference produced between a first electrode and a second electrode brought into contact with a body surface. The first electrode is provided on an outer surface of a device main body, and the second electrode is drawn outside the device main body via a connection cable.

Preferably, in the portable electrocardiograph according to the present invention, the device main body has a substantially rectangular parallelepiped shape, and the first electrode is provided on an end surface located at one end in a longitudinal direction of the device main body.

Preferably, the connection cable is attached to the device main body in a detachable manner.

Preferably, in the portable electrocardiograph according to the present invention, the device main body includes wind-up means for winding up the connection cable in the device main body.

Preferably, in the portable electrocardiograph according to the present invention, the device main body includes a storage space accommodating the connection cable and the second electrode.

Preferably, in the portable electrocardiograph according to the present invention, the first electrode is to be brought into contact with a right hand, and the second electrode is to be brought into contact with a chest.

Preferably, in the portable electrocardiograph according to the present invention, the first electrode is to be brought into contact with a chest while the device main body is held with a left hand, and the second electrode is to be brought into contact with a right hand.

According to the present invention, superposition on the electrocardiographic waveform of the noise caused by the myoelectric potential produced in muscles other than cardiac muscle is avoided, thereby allowing precise measurement of the electrocardiographic waveform. Therefore, the present invention contributes to early detection of ischemic cardiomyopathies or the like, and allows proper diagnosis.

The foregoing and other objects, features, aspects and advantages of the present invention will become more apparent from the following detailed description of the present invention when taken in conjunction with the accompanying drawings.

### BRIEF DESCRIPTION OF THE DRAWINGS

Fig. 1 is a perspective view of an appearance of a portable electrocardiograph in a first embodiment of the present invention.
Fig. 2 is a front view of the portable electrocardiograph in the first embodiment of the present invention.
Fig. 3 is a top view of the portable electrocardiograph in the first embodiment of the present invention.
Fig. 4 is a bottom view of the portable electrocardiograph in the first embodiment of the present invention.
Fig. 5 is a right side view of the portable electrocardiograph in the first embodiment of the present invention.
Fig. 6 is a left side view of the portable electrocardiograph in the first embodiment of the present invention.
Fig. 7 is a perspective view of a measurement posture to be taken by a subject at the time of measuring an electrocardiographic waveform using the portable electrocardiograph in the first embodiment of the present invention.
Fig. 8 illustrates a measurement posture to be taken by the subject at the time of measuring an electrocardiographic waveform using the portable electrocardiograph in the first embodiment of the present invention, viewed from the above.
Fig. 9 illustrates a state where a device main body is held by the subject at the time of measuring an electrocardiographic waveform using the portable electrocardiograph in the first embodiment of the present invention.
Fig. 10 is a partially cut-away side view of another structural example of an external electrode that can be employed in the portable electrocardiograph in the first embodiment of the present invention.
Fig. 11 is a side view of yet another structural example of the external electrode that can be employed in the portable electrocardiograph in the first embodiment of the present invention.
Fig. 12 is a perspective view of an appearance of a portable electrocardiograph in a second embodiment of the present invention.
Fig. 13 is a front view of the portable electrocardiograph in the second embodiment of the present invention.
Fig. 14 is a top view of the portable electrocardiograph in the second embodiment of the present invention.
Fig. 15 is a bottom view of the portable electrocardiograph in the second embodiment of the present invention.
Fig. 16 is a right side view of the portable electrocardiograph in the second embodiment of the present invention.
Fig. 17 is a left side view of the portable electrocardiograph in the second embodiment of the present invention.
Fig. 18 is a perspective view of a measurement posture to be taken by a subject at the time of measuring an electrocardiographic waveform using the portable electrocardiograph in the second embodiment of the present invention.
Fig. 19 illustrates a measurement posture to be taken by the subject at the time of measuring an electrocardiographic waveform using the portable electrocardiograph in the second embodiment of the present invention, viewed from the above.
Fig. 20 illustrates a state where a device main body is held by the subject at the time of measuring an electrocardiographic waveform using the portable electrocardiograph in the second embodiment of the present invention.
Fig. 21 is a side view of another structural example of the external electrode that can be employed in the portable electrocardiograph in the second embodiment of the present invention.
Fig. 22 is a side view of yet another structural example of the external electrode that can be employed in the portable electrocardiograph in the second embodiment of the present invention.
Fig. 23 is a perspective view of another example of the measurement posture to be taken by the subject at the time of measuring an electrocardiographic waveform using the portable electrocardiograph in the second embodiment of the present invention.
Fig. 24 is a schematic perspective view of a structure of a portable electrocardiograph in a third embodiment of the present invention.
Fig. 25 illustrates a variation of the portable electrocardiograph in the third embodiment of the present invention.
Fig. 26 is a schematic perspective view of a structure of a portable electrocardiograph in a fourth embodiment of the present invention.
Fig. 27 is a perspective view of an example of a conventional portable electrocardiograph.
Fig. 28A is a front view of another example of the conventional portable electrocardiograph.
Fig. 28B is a left side view of the conventional portable electrocardiograph illustrated in Fig. 28A.
Fig. 28C is a left side view illustrating a state where the conventional portable electrocardiograph shown in Figs. 28A and 28B is opened.
Fig. 29A is a front view of yet another example of the conventional portable electrocardiograph.
Fig. 29B is a right side view of the conventional portable electrocardiograph illustrated in Fig. 29A.
Figs. 30 and 31 are front views of yet other examples of the conventional portable electrocardiograph.
Fig. 32 illustrates a measurement posture to be taken by the subject at the time of measuring an electrocardiographic waveform using the portable electrocardiograph illustrated in Fig. 30.
Fig. 33 illustrates a holding state with a right hand at the time of measuring an electrocardiographic waveform using the portable electrocardiograph illustrated in Fig. 30.

### DESCRIPTION OF THE PREFERRED EMBODIMENTS

In the following, embodiments of the present invention will be described in detail with reference to the drawings.

### (First Embodiment)

First, an overall structure of a portable electrocardiograph 100A in the present embodiment will be described.

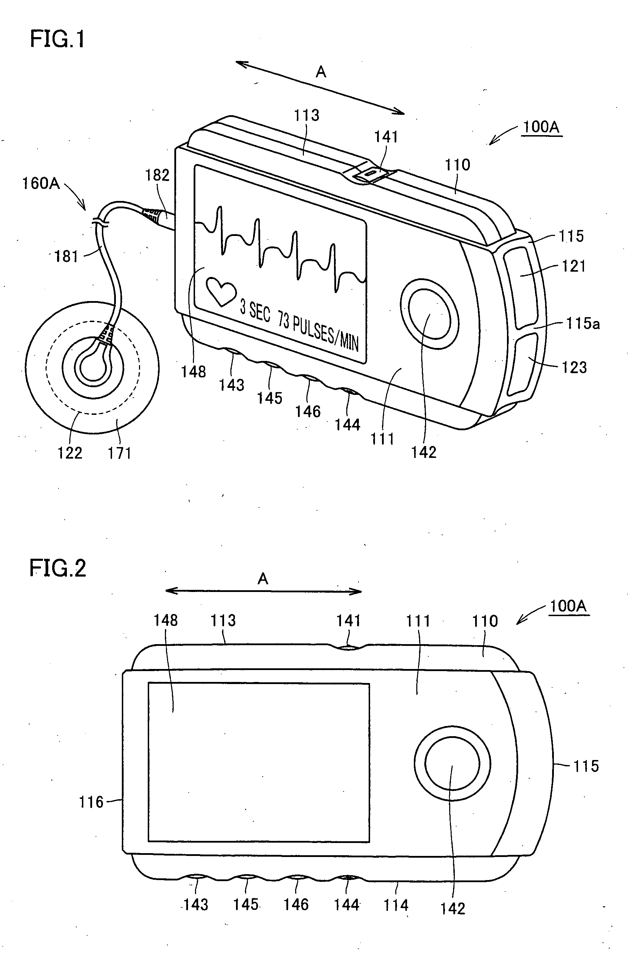

As shown in Figs. 1 to 6, in order to realize excellent usability, portable electrocardiograph 100A in the present embodiment has such a light weight and a small size that it can be held by one hand. Portable electrocardiograph 100A includes a device main body 110 and an external electrode unit 160A.

A structure of device main body 110 will now be described. Device main body 110 has a flat and elongated, substantially rectangular parallelepiped shape. On its outer surfaces (front face 111, rear face 112, top face 113, bottom face 114, right side face 115, and left side face 116), a display unit, an operation unit, electrodes and the like are disposed.

As shown in Figs. 1 and 2, a measurement button 142 serving as an operation button for starting measurement is provided in a portion close to one end in the longitudinal direction (the direction shown with an arrow A in the drawing) of front face 111 of device main body 110. In a portion close to the other end of front face 111 of device main body 110, display unit 148 is provided. Display unit 148 is implemented, for example, by a liquid crystal display and serves to display a result of measurement or the like. The measurement result is displayed, for example, as electrocardiographic waveforms or numerical data as shown in Fig. 1.

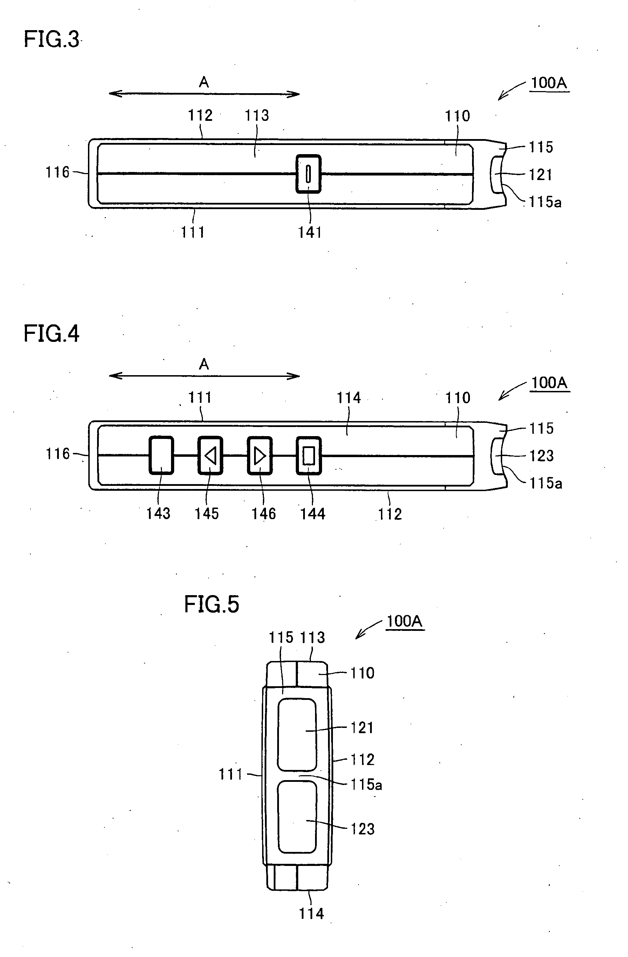

As shown in Figs. 1 and 3, power button 141 is disposed in a prescribed position in top surface 113 of device main body 110. Power button 141 serves as an operation button for turning ON/OFF portable electrocardiograph 100A.

As shown in Figs. 1 and 4, various operation buttons are disposed in prescribed positions on bottom face 114 of device main body 110. In shown portable electrocardiograph 100A, a setting button 143, a display button 144, a left scroll button 145, and a right scroll button 146 are disposed. Setting button 143 is used for making a variety of settings for portable electrocardiograph 100A, while display button 144 is used for displaying a measurement result on display unit 148. Left scroll button 145 and right scroll button 146 are used for scrolled display of a graph showing a measurement result or guide information displayed on display unit 148.

As shown in Figs. 1 and 5, on right side face 115 located at one end in the longitudinal direction of device main body 110, first electrode (negative electrode) 121 representing one electrode out of a pair of measurement electrodes as well as indifferent electrode 123 for deriving a potential serving as a reference in potential variation in the body are disposed. Right side face 115 has a smoothly curved shape, such that a forefinger of the right hand of the subject is fitted thereto when the subject takes a measurement posture which will be described later. In addition, a concave portion 115a extending in an up-down direction is formed in right side face 115. Concave portion 115a is in a shape to receive the forefinger of the right hand of the subject.

First electrode 121 and indifferent electrode 123 described above are formed with a conductive member, and electrically connected to a circuit formed inside device main body 110. In addition, first electrode 121 and indifferent electrode 123 are disposed in concave portion 115a provided in right side face 115 such that their surfaces are exposed on the outer surface of device main body 110. The first electrode 121 is located closer to top face 113 on right side face 115, while indifferent electrode 123 is located closer to bottom face 114 on right side face 115.

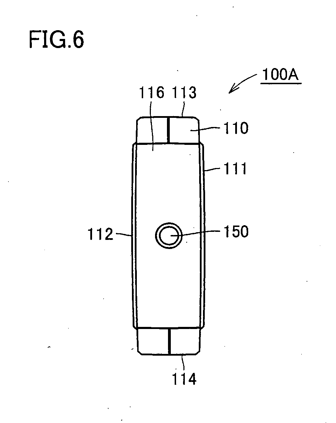

As shown in Fig. 6, on left side face 116 of device main body 110, a jack 150 receiving connector 182 (see Fig. 1) provided at the tip end of connection cable 181 of external electrode unit 160A which will be described later is provided. Jack 150 serves as a connection terminal for electrically connecting second electrode 122 (see Fig. 1) which will be described later to the circuit provided inside device main body 110.

A structure of external electrode unit 160A will now be described. As shown in Fig. 1, external electrode unit 160A includes attachment portion 171, connection cable 181, and connector 182. Attachment portion 171 represents a part for attaching second electrode 122 which will be described later to the body. In portable electrocardiograph 100A in the present embodiment, what is called an adhesion-type electrode is adopted. On a main surface of attachment portion 171, second electrode (positive electrode) 122 representing the other electrode of the pair of measurement electrodes is disposed. A conductive adhesive is applied to the surface of second electrode 122, and second electrode 122 is attached to the body by means of the adhesive layer.

Connection cable 181 has one end electrically connected to second electrode 122, and has the other end connected to connector 182. Preferably, a highly flexible connection cable 181 is used, from the viewpoint of ease of use. Connector 182 is detachably inserted in jack 150 provided in device main body 110 described above. When connector 182 is inserted in jack 150, second electrode 122 provided in external electrode unit 160A is electrically connected to the circuit provided inside device main body 110.

Next, a measurement posture to be taken by the subject in measuring an electrocardiographic waveform using portable electrocardiograph 100A having the above-described structure will be described.

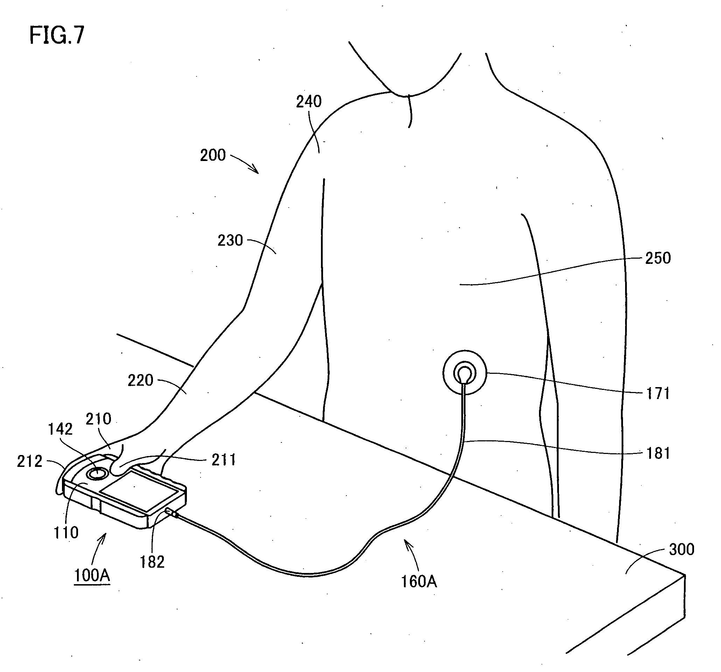

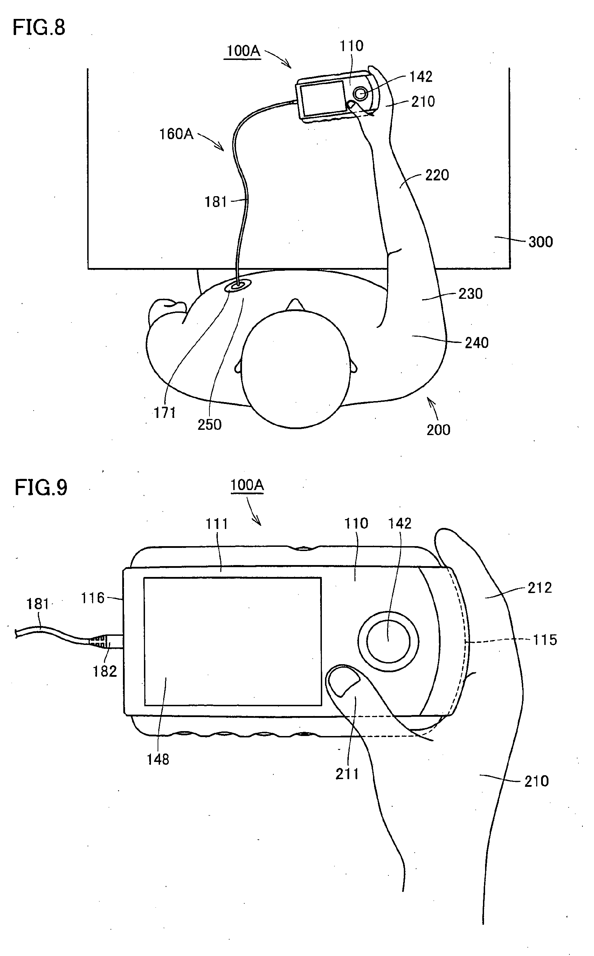

As shown in Figs. 7 and 8, during measurement, a state in which connector 182 of external electrode unit 160A is inserted in jack 150 of device main body 110 is maintained. Subject 200 presses down power button 141 provided on top face 113 of device main body 110, so as to turn on portable electrocardiograph 100A.

Then, the subject holds a portion closer to one end in the longitudinal direction of device main body 110 such that right side face 115 of device main body 110 is covered with forefinger 212 of right hand 210. Right forearm 220 is placed on a base such as a desk 300. Here, subject 200 preferably sits on a chair or the like, and takes a relaxed posture without imposing burden on the body.

Thereafter, attachment portion 171 of external electrode unit 160A is stuck to chest 250. More specifically, attachment portion 171 is stuck such that second electrode 122 provided in external electrode unit 160A comes in contact with the skin on a fifth intercostal anterior axillary line of chest 250. Then, the subject presses measurement button 142 provided on front face 111 of device main body 110 with his/her thumb 211 of right hand 210 holding device main body 110, and maintains the measurement posture at ease for several tens of seconds until measurement of the electrocardiographic waveform is completed.

A state that portable electrocardiograph 100A is held with right hand 210 will now be described.

As shown in Fig. 9, in this measurement posture, subject 200 holds with right hand 210 a portion closer to one end in the longitudinal direction of device main body 110 such that front face 111 of device main body 110 of portable electrocardiograph 1 00A faces upward. Here, right side face 115 of device main body 110 is covered with forefinger 212 of right hand 210, thumb 211 of right hand 210 is placed on front face 111 of device main body 110, and the middle finger of right hand 210 is placed on the rear face of device main body 110. That is, device main body 110 is held such that it is caught by three fingers.

In this state, forefinger 212 of right hand 210 is lightly bent such that the forefinger extends along curved right side face 115 and is inserted in concave portion 115a provided in right side face 115. Forefinger 212 of right hand 210 is thus brought into contact with first electrode 121 and indifferent electrode 123 provided in concave portion 115a.

When such a measurement posture is taken, first electrode 121 and indifferent electrode 123 provided on the outer surface of device main body 110 of portable electrocardiograph 100A come in contact with forefinger 212 of right hand 210 of subject 200, and second electrode 122 connected to device main body 110 via connection cable 181 comes in contact with chest 250 of subject 200. In this manner, a measurement circuit is implemented by right hand 210 being in contact with first electrode 121, forearm 220 without contacting chest 250, a brachium 230 and a right shoulder 240 without contacting chest 250, and chest 250 to which second electrode 122 is attached, in this order.

According to portable electrocardiograph 100A in the present embodiment, a potential difference produced between first electrode 121 provided on the outer surface of device main body 110 and second electrode 122 drawn outside device main body 110 via connection cable 181 is measured, so as to measure the electrocardiographic waveform. With such a structure, the measurement posture as shown in Figs. 7 and 8 can be allowed.

In the measurement posture shown in Figs. 7 and 8, right forearm 220 is placed on the base such as desk 300. Accordingly, subject 200 does not have to put unnecessary strength into his/her right arm, and the electrocardiographic waveform can be taken with the subject taking a very relaxed posture. The muscles in the right arm are not under tension, and generation of the myoelectric potential is effectively suppressed. Consequently, noise caused by the myoelectric potential produced in the muscles in the right arm is not superposed on obtained measurement data, whereby the electrocardiographic waveform can precisely be measured in a stable manner.

In addition, according to portable electrocardiograph 100A in the present embodiment, first electrode 121 provided on the outer surface of device main body 110 is provided on right side face 115 located at one end in the longitudinal direction of device main body 110. Accordingly, when the electrocardiographic waveform is measured while the measurement posture as shown in Figs. 7 and 8 is taken, display unit 148 can visually be recognized during measurement. That is, measurement while checking the measurement data can be achieved.

Moreover, according to portable electrocardiograph 100A in the present embodiment, external electrode unit 160A including connection cable 181 is attached to device main body 110 in a detachable manner, thereby portability being improved.

Though the external electrode unit including the adhesion-type electrode has been described by way of example in the present embodiment, the external electrode unit connected to the device main body is not necessarily limited to the above-described type. For example, an external electrode unit including a suction-cup-type electrode or an external electrode unit including a pressed-type electrode may be adopted.

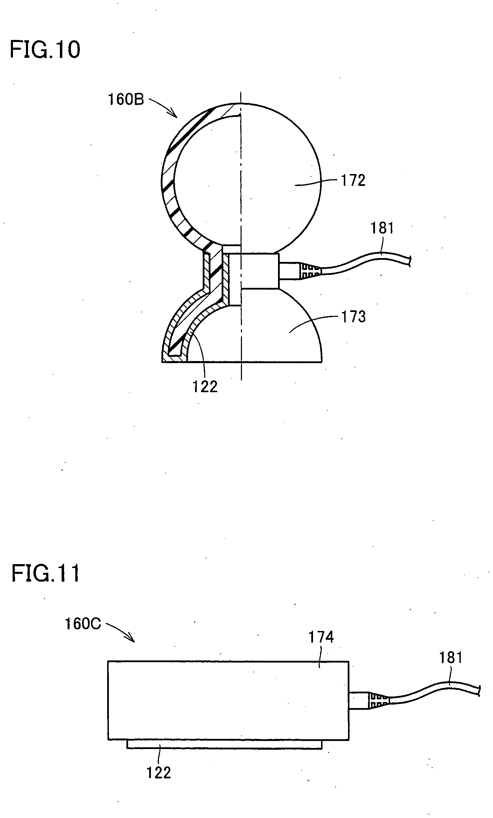

An external electrode unit 160B shown in Fig. 10 represents an external electrode unit including what is called a suction-cup-type electrode, and includes a negative pressure creating portion 172 and a suction cup portion 173. Negative pressure creating portion 172 and suction cup portion 173 are formed, for example, with a rubber material, and suction cup portion 173 is covered with a conductive coating. The conductive coating implements second electrode 122, and it is electrically connected to connection cable 181.

In attaching the suction-cup-type electrode to the body, negative pressure creating portion 172 is pinched by fingers, so as to compress an internal space. Then, suction cup portion 173 is pressed against a prescribed site of the body, and the fingers that have been pinched negative pressure creating portion 172 are removed. Then, a negative pressure is caused in the internal space, and the suction-cup-type electrode is stuck and held to the body. When the external electrode unit including such a suction-cup-type electrode is employed as well, the electrocardiographic waveform can precisely be measured in a stable manner, as in employing the external electrode unit including the adhesion-type electrode.

An external electrode unit 160C shown in Fig. 11 represents an external electrode unit including what is called a pressed-type electrode, and includes a base body 174 and second electrode 122 provided on its outer surface. Second electrode 122 is formed with a conductive member, and electrically connected to connection cable 181.

In bringing the pressed-type electrode into contact with the body, base body 174 is held with a left hand and pressed against a prescribed site of the body. When the external electrode unit including such a pressed-type electrode is employed as well, the electrocardiographic waveform can precisely be measured in a stable manner, as in employing the external electrode unit including the adhesion-type electrode.

### (Second Embodiment)

An overall structure of a portable electrocardiograph 100B in the present embodiment will now be described.

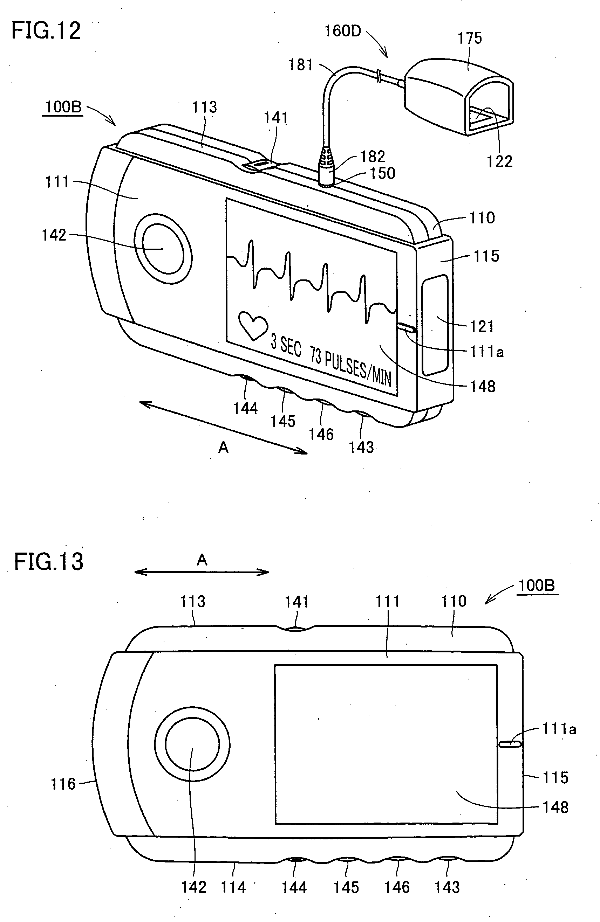

As shown in Figs. 12 to 17, in order to realize excellent usability, portable electrocardiograph 1 00B in the present embodiment has such a light weight and a small size that it can be held by one hand, as in the portable electrocardiograph in the first embodiment described above. Portable electrocardiograph 100B includes device main body 110 and an external electrode unit 160D.

A structure of device main body 110 will now be described. Device main body 110 has a flat and elongated, substantially rectangular parallelepiped shape. On its outer surfaces (front face 111, rear face 112, top face 113, bottom face 114, right side face 115, and left side face 116), a display unit, an operation unit, electrodes and the like are disposed.

As shown in Figs. 12 and 13, display unit 148 is provided in a portion close to one end in the longitudinal direction (the direction shown with arrow A in the drawing) of front face 111 of device main body 110. Display unit 148 is implemented, for example, by a liquid crystal display and serves to display a result of measurement or the like. The measurement result is displayed, for example, as electrocardiographic waveforms or numerical data as shown in Fig. 12. In a portion close to the other end of front face 111 of device main body 110, measurement button 142 serving as an operation button for starting measurement is provided. In an end portion closer to one end of front face 111 of device main body 110, an alignment mark 111a serving as an index in pressing first electrode 121 which will be described later against the body is formed.

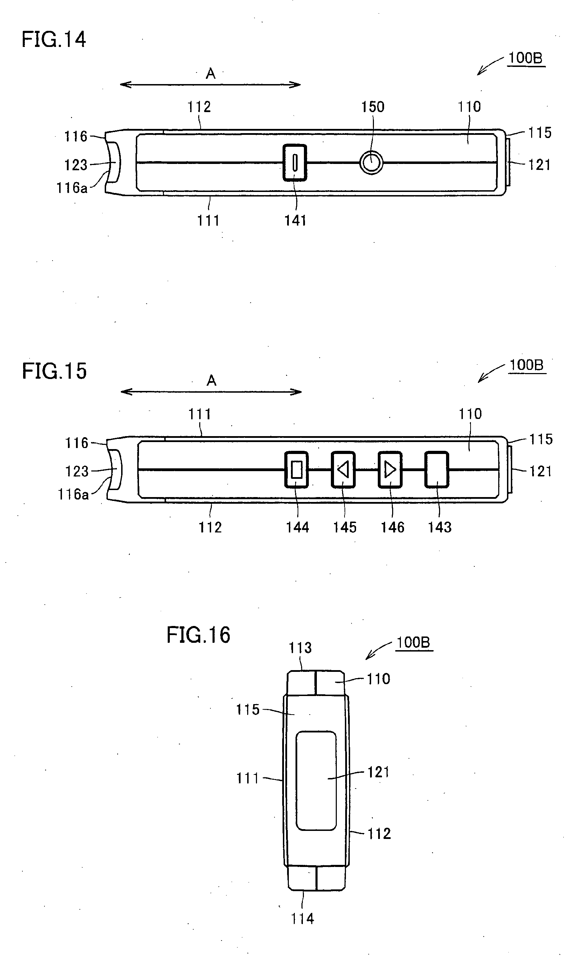

As shown in Figs. 12 and 14, power button 141 is disposed in a prescribed position in top surface 113 of device main body 110. Power button 141 serves as an operation button for turning ON/OFF portable electrocardiograph 100B. In a prescribed position on top face 113 of device main body 110, jack 150 receiving connector 182 (see Fig. 12) provided at the tip end of connection cable 181 of external electrode unit 160D which will be described later is provided. Jack 150 serves as a connection terminal for electrically connecting second electrode 122 (see Fig. 12) which will be described later to a circuit provided inside device main body 110.

As shown in Figs. 12 and 15, various operation buttons are disposed in prescribed positions on bottom face 114 of device main body 110. In shown portable electrocardiograph 100B, setting button 143, display button 144, left scroll button 145, and right scroll button 146 are disposed. Setting button 143 is used for making a variety of settings for portable electrocardiograph 100B, while display button 144 is used for displaying the measurement result on display unit 148. Left scroll button 145 and right scroll button 146 are used for scrolled display of a graph showing a measurement result or guide information displayed on display unit 148.

As shown in Figs. 12 and 16, on right side face 115 located at one end in the longitudinal direction of device main body 110, first electrode (positive electrode) 121 representing one electrode out of a pair of measurement electrodes is disposed. First electrode 121 is formed with a conductive member, and electrically connected to the circuit provided inside device main body 110.

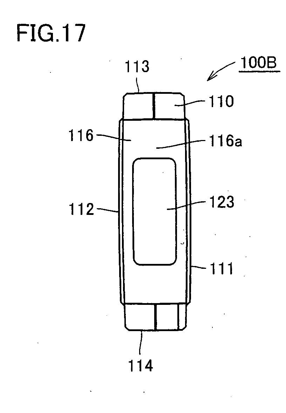

As shown in Fig. 17, on left side face 116 located at the other end in the longitudinal direction of device main body 110, indifferent electrode 123 for deriving a potential serving as a reference in potential variation in the body is disposed. Indifferent electrode 123 is formed with a conductive member, and electrically connected to the circuit provided inside device main body 110.

Left side face 116 has a smoothly curved shape, such that a forefinger of the left hand of the subject is fitted thereto when the subject takes a measurement posture which will be described later. In addition, a concave portion 116a extending in an up-down direction is formed in left side face 116. Concave portion 116a is in a shape to receive the forefinger of the left hand of the subject.

A structure of external electrode unit 160D will now be described. As shown in Fig. 12, external electrode unit 160D includes a base body 175, connection cable 181, and connector 182. Base body 175 is made of a box-shaped member having an opening in one surface, and includes second electrode (negative electrode) 122 on its inner bottom face. Base body 175 has such a shape as to receive in its inner space the forefinger of the right hand through the opening. Base body 175 is structured such that when the forefinger of the right hand is inserted therein, the forefinger tip comes in contact with second electrode 122.

Connection cable 181 has one end electrically connected to second electrode 122, and has the other end connected to connector 182. Preferably, a highly flexible connection cable 181 is used, from the viewpoint of ease of use. Connector 182 is detachably inserted in jack 150 provided in device main body 110 described above. When connector 182 is inserted in jack 150, second electrode 122 provided in external electrode unit 160D is electrically connected to the circuit provided inside device main body 110.

Next, a measurement posture to be taken by the subject in measuring an electrocardiographic waveform using portable electrocardiograph 100B having the above-described structure will be described.

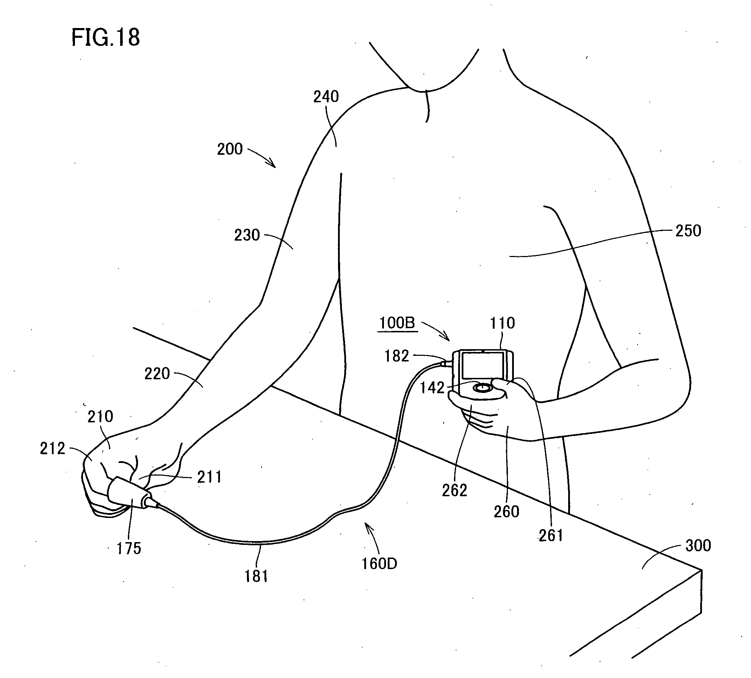

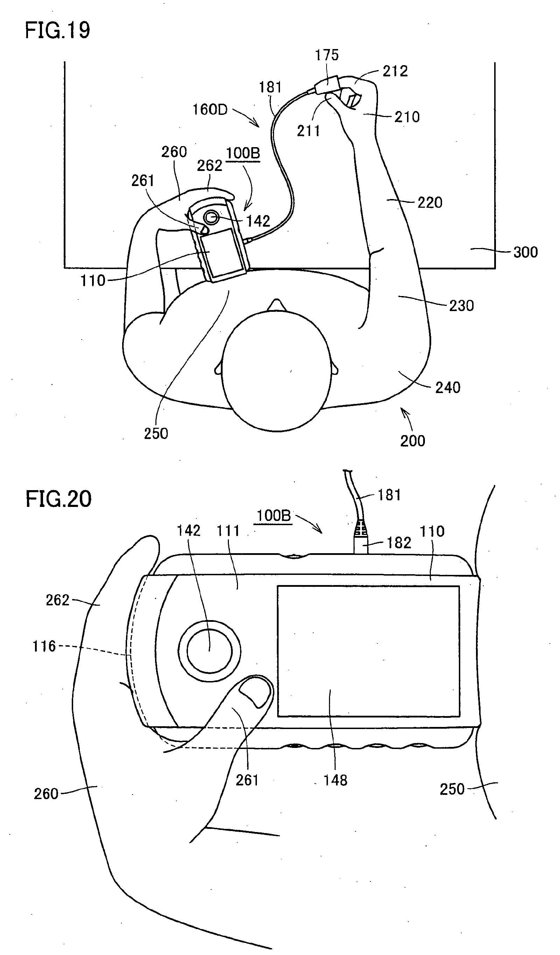

As shown in Figs. 18 and 19, during measurement, a state in which connector 182 of external electrode unit 160D is inserted in jack 150 of device main body 110 is maintained. Subject 200 presses down power button 141 provided on top face 113 of device main body 110, so as to turn on portable electrocardiograph 100B.

Then, forefinger 212 of right hand 210 is inserted in the inner space of base body 175 of external electrode unit 160D, so as to bring forefinger 212 into contact with second electrode 122. Right forearm 220 is placed on the base such as desk 300. Here, preferably, thumb 211 of right hand 210 is brought into contact with a rear face of base body 175. In this manner, base body 175 is held by forefinger 212 and thumb 211 in a stable manner, and contact between second electrode 122 and forefinger 212 of right hand 210 is maintained in a stable manner.

Then, the subject holds a portion closer to the other end in the longitudinal direction of device main body 110 such that left side face 116 of device main body 110 is covered with a forefinger 262 of a left hand 260, and first electrode 121 provided on right side face 115 of device main body 110 is brought in direct contact with the skin on the fifth intercostal anterior axillary line located in a lower left portion of chest 250. Here, subject 200 preferably sits on a chair or the like, and takes a relaxed posture without imposing burden on the body. Then, the subject presses measurement button 142 provided on front face 111 of device main body 110 with a thumb 261 of left hand 260 holding device main body 110, and the subject maintains this measurement posture at ease for several tens of seconds until measurement of the electrocardiographic waveform is completed.

A state that portable electrocardiograph 100B is held with left hand 260 will now be described.

As shown in Fig. 20, in this measurement posture, subject 200 holds a portion closer to the other end in the longitudinal direction of device main body 110 with left hand 260 such that front face 111 of device main body 110 of portable electrocardiograph 100B faces upward. Here, left side face 116 of device main body 110 is covered with forefinger 262 of left hand 260, thumb 261 of left hand 260 is placed on front face 111 of device main body 110, and the middle finger of left hand 260 is placed on the rear face of device main body 110. That is, device main body 110 is held such that it is caught by three fingers.

In this state, forefinger 262 of left hand 260 is lightly bent such that the forefinger extends along curved left side face 116 and is inserted in concave portion 116a provided in left side face 116. Forefinger 262 of left hand 260 is thus brought into contact with indifferent electrode 123 provided in concave portion 116a.

When such a measurement posture is taken, first electrode 121 provided on right side face 115 of device main body 110 of portable electrocardiograph 100B comes in contact with chest 250 of subject 200, and indifferent electrode 123 provided on left side face 116 of device main body 110 comes in contact with forefinger 262 of left hand 260 of subject 200. In addition, second electrode 122 connected to device main body 110 via connection cable 181 comes in contact with forefinger 212 of right hand 210 of subject 200. In this manner, a measurement circuit is implemented by right hand 210 being in contact with second electrode 122, forearm 220 without contacting chest 250, brachium 230 and right shoulder 240 without contacting chest 250, and chest 250 to which first electrode 121 is attached, in this order.

According to portable electrocardiograph 100B in the present embodiment, a potential difference produced between first electrode 121 provided on the outer surface of device main body 110 and second electrode 122 drawn outside device main body 110 via connection cable 181 is measured, so as to measure the electrocardiographic waveform. With such a structure, the measurement posture as shown in Figs. 18 and 19 can be allowed.

In the measurement posture shown in Figs. 18 and 19, right forearm 220 is placed on the base such as desk 300. Accordingly, subject 200 does not have to put unnecessary strength into his/her right arm, and the electrocardiographic waveform can be taken with the subject taking a very relaxed posture. The muscles in the right arm are not under tension, and generation of the myoelectric potential is effectively suppressed. Consequently, noise caused by the myoelectric potential produced in the muscles in the right arm is not superposed on obtained measurement data, whereby the electrocardiographic waveform can precisely be measured in a stable manner. Here, device main body 110 of portable electrocardiograph 100B is held with the left hand. Therefore, the myoelectric potential produced in muscles in the left arm does not appear on the measurement circuit. That is, noise caused by the myoelectric potential is not superposed on the obtained electrocardiographic waveform.

In addition, according to portable electrocardiograph 100B in the present embodiment, first electrode 121 provided on the outer surface of device main body 110 is provided on right side face 115 located at one end in the longitudinal direction of device main body 110. Accordingly, the measurement posture to allow the measurement circuit to cross over the heart is realized in an ensured manner, and short-circuit of the measurement circuit due to contact of right hand 210, forearm 220 and brachium 230 with chest 250 is prevented. When the electrocardiographic waveform is measured while the measurement posture as shown in Figs. 18 and 19 is taken as well, display unit 148 can visually be recognized during measurement. That is, measurement while checking the measurement data can be achieved.

Moreover, according to portable electrocardiograph 100B in the present embodiment, external electrode unit 160D including connection cable 181 is attached to device main body 110 in a detachable manner, thereby portability being improved.

Though the external electrode unit including the electrode receiving the right forefinger has been described by way of example in the present embodiment, the external electrode unit connected to the device main body is not necessarily limited to the above-described type. For example, an external electrode unit including a grip-type electrode or an external electrode unit including a pinched-type electrode may be adopted. In addition, though portable electrocardiograph 100B in the present embodiment has indifferent electrode 123 disposed on left side face 116 of device main body 110, indifferent electrode 123 may be provided in the external electrode unit instead of being disposed on device main body 110, as will be described later.

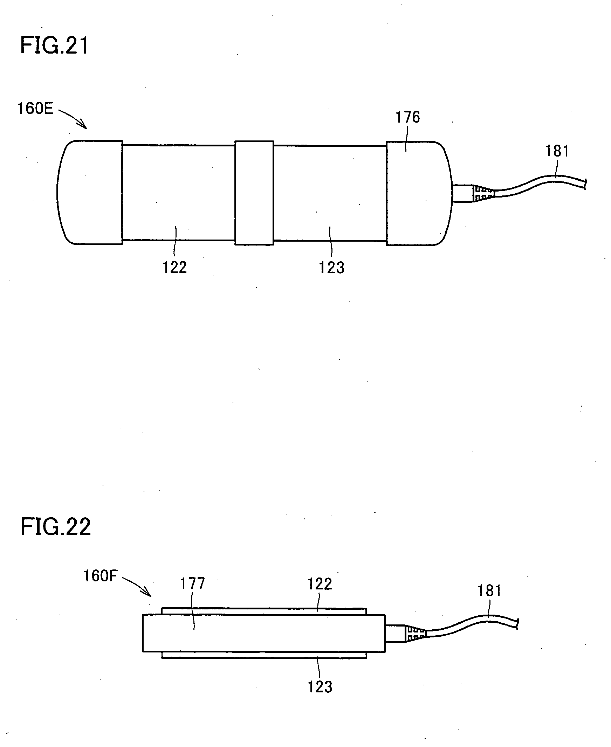

An external electrode unit 160E shown in Fig. 21 represents an external electrode unit including what is called a grip-type electrode, and includes a base body 176 as well as second electrode 122 and indifferent electrode 123 provided on its outer surface. Second electrode 122 and indifferent electrode 123 are formed with a conductive member, and electrically connected to connection cable 181.

In contacting the grip-type electrode with the body, base body 176 is gripped by the right hand. During measurement, attention should be paid not to release the hand. When the external electrode unit including such a grip-type electrode is employed as well, the electrocardiographic waveform can precisely be measured in a stable manner.

An external electrode unit 160F shown in Fig. 22 represents an external electrode unit including a pinched-type electrode, and includes a base body 177 as well as second electrode 122 and indifferent electrode 123 provided on its outer surface. Second electrode 122 and indifferent electrode 123 are formed with a conductive member, and provided on opposing main surfaces of base body 177 respectively. Second electrode 122 and indifferent electrode 123 are electrically connected to connection cable 181.

In contacting the pinched-type electrode with the body, for example, the thumb of the right hand is brought into contact with indifferent electrode 123, the forefinger and the middle finger of the right hand are brought into contact with second electrode 122. In doing so, base body 177 is held with the right hand, and attention should be paid not to release the hand during measurement. When the external electrode unit including such a pinched-type electrode is employed as well, the electrocardiographic waveform can precisely be measured in a stable manner.

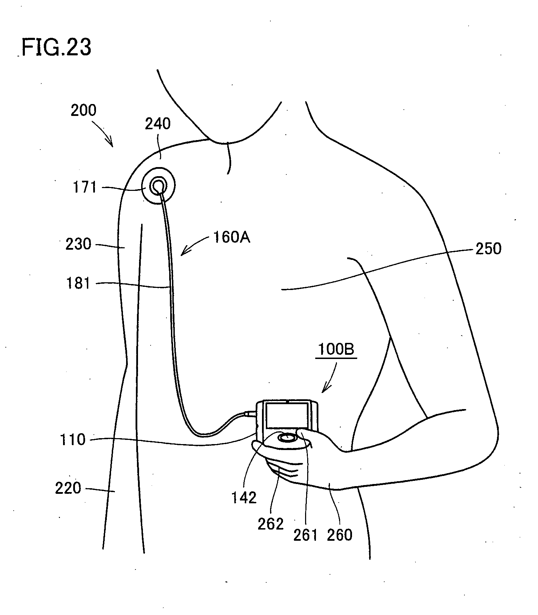

Portable electrocardiograph 100B in the present embodiment may adopt external electrode unit 160A including what is called the adhesion-type electrode described in the first embodiment above. Fig. 23 shows a measurement posture in such a case.

As shown in Fig. 23, when external electrode unit 160A including the adhesion-type electrode is employed, the device main body is held and pressed against the body with the left hand in a manner the same as in the measurement posture shown in Fig. 18 as set forth above. Meanwhile, it is preferable to change a position where the adhesion-type electrode provided in external electrode unit 160A contacts with the body. That is, the electrode is preferably attached to the right shoulder in measuring the electrocardiographic waveform. In this manner, second electrode 122, right shoulder 240, chest 250, and first electrode 121 implement the measurement circuit in this order. Accordingly, regardless of a state of the right arm, superposition on the electrocardiographic waveform of the noise caused by the myoelectric potential produced in the muscles in the right arm is considerably suppressed. As a result, more precise measurement of the electrocardiographic waveform can be achieved.

### (Third Embodiment)

A portable electrocardiograph 100C in the present embodiment is intended to measure the electrocardiographic waveform in the measurement posture shown in Figs. 7 and 8, in a manner similar to portable electrocardiograph 100A in the first embodiment described above. Therefore, portions the same as those in the first embodiment described above are given the same reference characters in the drawings, and description thereof will not repeated.

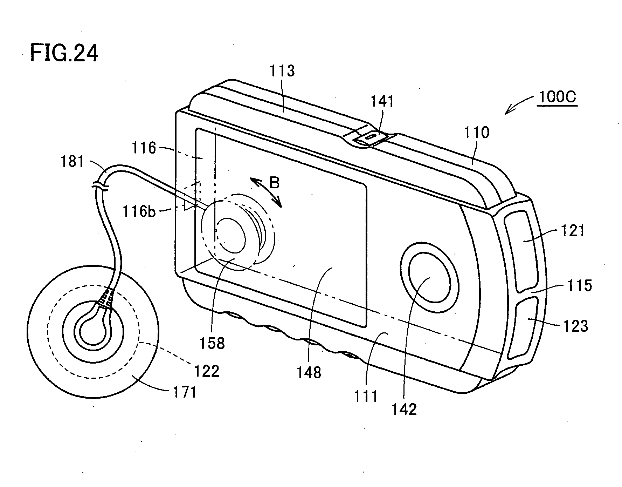

As shown in Fig. 24, portable electrocardiograph 100C in the present embodiment includes a cord reel 158 serving as wind-up means for winding up connection cable 181 of external electrode unit 160A in device main body 110. Connection cable 181 wound on cord reel 158 is drawn outside device main body 110 through an opening 116b provided in left side face 116 of device main body 110, and its tip end is electrically connected to second electrode 122 provided in external electrode unit 160A. Cord reel 158 turns in a direction shown with an arrow B in the drawing, so as to freely adjust a length of connection cable 181.

According to such a structure, not only the length of connection cable 181 can be adjusted, but also connection cable 181 is wound by means of cord reel 158 during a period in which measurement is not performed. Accordingly, a portable electrocardiograph attaining excellent portability can be obtained.

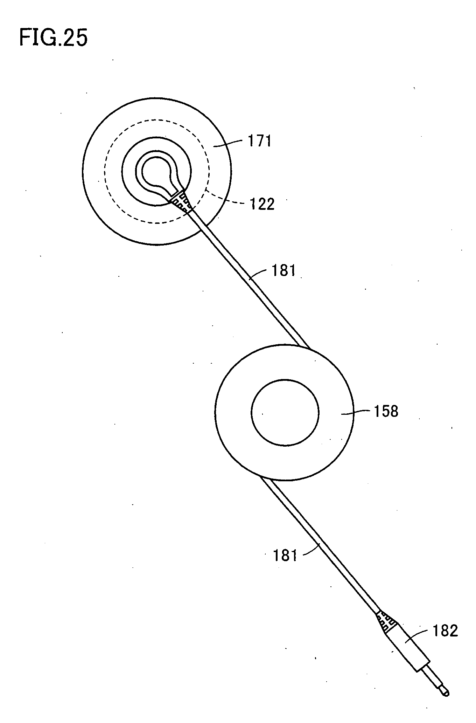

As shown in Fig. 25, cord reel 158 may be provided in the external electrode unit. In such a case, cord reel 158 is attached to some midpoint of connection cable 181.

### (Fourth Embodiment)

A portable electrocardiograph 100D in the present embodiment is intended to measure the electrocardiographic waveform in the measurement posture shown in Figs. 7 and 8, in a manner similar to portable electrocardiograph 100A in the first embodiment described above. Therefore, portions the same as those in the first embodiment described above are given the same reference characters in the drawings, and description thereof will not repeated.

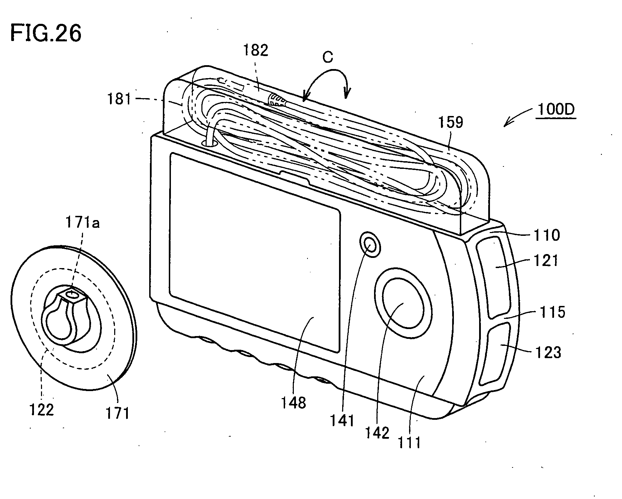

As shown in Fig. 26, portable electrocardiograph 100D in the present embodiment includes a storage space for accommodating connection cable 181 of external electrode unit 160A on top face 113 of device main body 110. More specifically, a cover 159 provided over top face 113 of device main body 110 is pivoted in a direction shown with an arrow C in the drawing, so as to permit take-out/storage of connection cable 181.

Connection cable 181 is directly drawn out from device main body 110, and has its tip end connected to connector 182. Connector 182 is inserted in a jack 171a provided in attachment portion 171 of the adhesion-type electrode, so as to be electrically connected to second electrode 122.

According to such a structure, connection cable 181 can be stored inside device main body 110 during a period in which measurement is not performed. Accordingly, a portable electrocardiograph attaining excellent portability can be obtained. In order to achieve further improvement in portability, a storage space for accommodating the adhesion-type electrode may be provided within device main body 110.

Though embodiments based on the present invention have been described above, the present invention is not limited to those embodiments. The present invention aims to provide one electrode out of the measurement electrodes in the device main body of the portable electrocardiograph and the other electrode in a manner drawn outside the device main body, in order to adopt a measurement posture without putting unnecessary strength into the right arm for the purpose of suppressing generation of a myoelectric potential produced in muscles in the right arm during measurement of an electrocardiographic waveform. Therefore, a structure other than those can be modified as appropriate. For example, a position where an indifferent electrode is disposed or a position where a display unit, an operation unit, or the like is disposed can be modified as appropriate.

With regard to a position where the electrode is attached to the body, it is preferable to attach the electrode to the position described in the embodiments above, considering the purpose of precisely measuring an electrocardiographic waveform in a stable manner. Measurement, however, can be performed also when the electrode is attached to a different position.

Although the present invention has been described and illustrated in detail, it is clearly understood that the same is by way of illustration and example only and is not to be taken by way of limitation, the spirit and scope of the present invention being limited only by the terms of the appended claims.

## Claims

1. A portable electrocardiograph measuring an electrocardiographic waveform by measuring a potential difference produced between a first electrode (121) and a second electrode (122) being in contact with a body surface, wherein
said first electrode (121) is provided on an outer surface of a device main body (110), and
said second electrode (122) is drawn outside said device main body (110) via a connection cable (181).

2. The portable electrocardiograph according to claim 1, wherein
said device main body (110) has a substantially rectangular parallelepiped shape, and
said first electrode (121) is provided on an end surface (115) located at one end in a longitudinal direction of said device main body (110).

3. The portable electrocardiograph according to claim 2, wherein
said first electrode (121) is to be brought into contact with a right hand, and
said second electrode (122) is to be brought into contact with a chest.

4. The portable electrocardiograph according to claim 2, wherein
said first electrode (121) is to be brought into contact with a chest while said device main body (110) is held with a left hand, and
said second electrode (122) is to be brought into contact with a right hand.

5. The portable electrocardiograph according to claim 1, wherein
said connection cable (181) is attached to said device main body (110) in a detachable manner.

6. The portable electrocardiograph according to claim 1, wherein
said device main body (110) includes wind-up means for winding up said connection cable (181) in said device main body (110).

7. The portable electrocardiograph according to claim 1, wherein
said device main body (110) includes a storage space accommodating said connection cable (181) and said second electrode (122).

8. The portable electrocardiograph according to claim 1, wherein
said first electrode (121) is to be brought into contact with a right hand, and
said second electrode (122) is to be brought into contact with a chest.

9. The portable electrocardiograph according to claim 1, wherein
said first electrode (121) is to be brought into contact with a chest while said device main body (110) is held with a left hand, and
said second electrode (122) is to be brought into contact with a right hand.
